# EUROPEAN PATENT SPECIFICATION

(11) **EP 4 429 688 B1**
(45) Date of publication and mention of the grant of the patent: **29.10.2025**
(21) Application number: 22817614.5
(22) Date of filing: 11.11.2022
(51) Int. Cl.: A61K 36/28, A61K 36/61, A61P 27/10, A61K 36/45, A61K 36/48, A61K 36/534, A61K 36/80, A61K 36/8998

(54) **COMPOSITIONS FOR TREATING PRESBYOPIA**
ZUSAMMENSETZUNGEN ZUR BEHANDLUNG VON PRESBYOPIE
COMPOSITIONS POUR LE TRAITEMENT DE LA PRESBYTIE

(30) Priority: 12.11.2021 EP 21383026
(43) Date of publication of application: 18.09.2024
(73) Proprietor: Creative Advanced Technology S.L., 08211 Castellar del Vallés (ES)
(72) Inventor: MONAGAS ASENSIO, Pedro, 08211 CASTELLAR DEL VALLÉS (ES)
(74) Representative: ZBM Patents - Zea, Barlocci & Markvardsen
(86) International application number: PCT/EP2022/081594
(87) International publication number: WO 2023/084014

(56) References cited:
- WO-A1-2014/199379
- WO-A1-2019/071211
- LI J: "Traditional Chinese medicine meridian patch for improving vision, comprises Saposhnikovia divaricata root, Arisaema cum bile, Asarum, Achyranthes, Notopterygium root, parasitic Loranthus, agilawood, elecampane and radix", WPI / 2017 CLARIVATE ANALYTICS,, vol. 2018, no. 52, 1 May 2018 (2018-05-01), XP002806402
- CHEN L: "Eye beautifying liquid used for treating e.g. eye diseases, is prepared by cleaning rhubarb root, Acorus calamus, spearmint, cacumen platycladi, folium callicarpae formosanae and semen cassiae, adding pure water, mixing and sterilizing", WPI / 2017 CLARIVATE ANALYTICS,, vol. 2019, no. 86, 11 October 2019 (2019-10-11), XP002806403
- RANA M. JAMOUS ET AL: "Antiobesity and Antioxidant Potentials of Selected Palestinian Medicinal Plants", EVIDENCE-BASED COMPLEMENTARY AND ALTERNATIVE MEDICINE, vol. 2018, 13 June 2018 (2018-06-13), US, pages 1 - 21, XP055734503, ISSN: 1741-427X, DOI: 10.1155/2018/8426752
- DATABASE WPI Week 201852, Derwent World Patents Index; AN 2018-36417E, XP002806402
- DATABASE WPI Week 201986, Derwent World Patents Index; AN 2019-87565Y, XP002806403

## Description

### Technical Field

The present invention belongs to the field of plant extracts. In particular, the invention relates to a combination of plant extracts, a process for its production, compositions comprising the combination and their use as a medicament. The combinations and compositions of the invention are particularly useful for treating and preventing presbyopia.

### Background Art

From the age of about 40 years, and in a sudden manner in most cases, people perceive a reduced capacity to read at close distances in low light conditions, which is often compensated by moving objects further away to see them clearly. One of the most frequent complaints in this regard is the impossibility to read in bed or the difficulty to sew under artificial light.

This condition of increasing minimum focal length in individuals with good unaided distance vision is called presbyopia, which is also known as old eye or reading blur. Because of its etiology, presbyopia affects hyperopes and emmetropes (people with no visual dysfunction) earlier, and its onset is delayed in the case of myopes.

The ciliary muscle inside the eye is responsible for modifying the curvature and thickness of the crystalline lens, the lens that serves to focus sharp images on the retina at any viewing distance. The closer the object is to the observer, the more the crystalline lens must curve and increase their thickness.

With age, this ciliary muscle loses its contraction capacity and at the same time the lens decreases its flexibility and, therefore, its accommodation capacity. As a result, closer objects begin to look blurry.

Currently there is no treatment that can correct presbyopia in the sense of restoring the accommodation capacity of the crystalline lens. All the methods currently used are based on refractive compensation or on lens substitution -the diopters that the crystalline lens cannot accommodate are provided by various methods, or the crystalline lens are replaced by synthetic ones.

In the majority of cases, presbyopia is compensated through the use of glasses or contact lenses. However, wearing glasses or lenses can be cumbersome and not suitable for everyone.

In very exceptional cases where the crystalline lens has to be removed due to cataracts, it can be replaced by multifocal intraocular lens that compensates the presbyopia. Naturally, this invasive solution is only suitable for a very particular subset of patients and involves several surgery-associated risks.

Rana M. Jamous et al., "Antiobesity and Antioxidant Potentials of Selected Palestinian Medicinal Plants" Alternative Medicine, vol. 2018, pp. 1-21, discloses individual plant extracts that present antioxidant and pancreatic lipase inhibition activity.

Thus, there is a need in the art for an effective and non-invasive treatment of presbyopia, in particular for a treatment that restores the accommodation capacity of the crystalline lens.

### Summary of Invention

The present inventor has developed a novel combination based on natural products for combating presbyopia. The combination of the invention comprises extracts of *Dittrichia viscosa, Syzygium aromaticum,* Vaccinium myrtillus, *Calendula officinalis, Euphrasia officinalis, Melilotus officinalis, Mentha spicata, Mimosa* tenuiflora and *Hordeum vulgare.*

Through extensive experimentation seeking natural compounds that could be useful for restoring the accommodation capacity of the crystalline lens in subjects suffering from presbyopia, the present inventor surprisingly found that the topical administration of a combination of *Dittrichia viscosa, Syzygium aromaticum,* Vaccinium myrtillus *Calendula officinalis, Euphrasia officinalis, Melilotus officinalis, Mentha spicata, Mimosa* tenuiflora and *Hordeum vulgare* extracts improved the vision at closed distances of presbyopic subjects.

As shown in the examples below, the inventor found that the ophthalmic administration of this particular combination of extracts improved all the near-vision parameters measured in twelve out of twelve subjects that received the combination (see table 2 of Example 3). Notably, the combination of the invention enhanced the near visual acuity, reduced near point of accommodation, and enhanced accommodative flexibility after only 7 days of treatment.

Without wishing to be bound by any theory, the present inventor believes that the therapeutic efficacy of the combination of the invention is based on its capacity to increase the elasticity and contraction capacity of the crystalline lens.

Hence, the combination of the invention herein provided represents a great step forward in the treatment of presbyopia. First of all, because it is capable of restoring the natural capacity of the crystalline lens to focus at close distances instead of simply compensating its dysfunction; secondly, the composition is based on safe and inexpensive natural products known to be well tolerated and secure for application in humans, thereby reducing the risk of undesired effects after long-term administration.

Thus, in a first aspect, the invention provides a combination comprising extracts of *Dittrichia viscosa, Syzygium aromaticum, Vaccinium myrtillus, Calendula officinalis, Euphrasia officinalis, Melilotus officinalis, Mentha spicata, Mimosa* tenuiflora and *Hordeum vulgare.*

In a second aspect, the invention provides a process for the production of the combination as defined in the first aspect, the process comprising the steps of (a) providing dry plant material of *Dittrichia viscosa, Syzygium aromaticum, Vaccinium myrtillus, Calendula officinalis, Euphrasia officinalis, Melilotus officinalis, Mentha spicata, Mimosa* tenuiflora and *Hordeum vulgare;* (b) boiling the dry plant material of (a) together in water, thereby obtaining a mixture; and (c) distilling the mixture of (b) so as to obtain the combination as defined in the first aspect; or, alternatively, (a) providing dry plant material of *Dittrichia viscosa, Syzygium aromaticum, Vaccinium myrtillus, Calendula officinalis, Euphrasia officinalis, Melilotus officinalis, Mentha spicata, Mimosa* tenuiflora and *Hordeum vulgare;* (b) boiling the dry plant material of (a) separately in water, thereby obtaining a plurality of mixtures; (b') distilling the plurality of mixtures of (b) thereby obtaining a plurality of extracts; and (c) combining the plurality of extracts of (b') so as to obtain the combination as defined in the first aspect; or, alternatively; (a) providing dry plant material of *Dittrichia viscosa, Syzygium aromaticum, Vaccinium myrtillus, Calendula officinalis, Euphrasia officinalis, Melilotus officinalis, Mentha spicata, Mimosa* tenuiflora and *Hordeum vulgare;*
(b) boiling the dry plant material of (a) separately in water, thereby obtaining a plurality of mixtures; (b') combining the plurality of mixtures of (b), thereby obtaining a single mixture; and (c) distilling the single mixture of (b') so as to obtain the combination as defined in the first aspect.

In a third aspect, the invention provides a combination as defined in the first aspect which is obtainable by the process as defined in the second aspect.

In a fourth aspect, the invention provides a composition comprising the combination as defined in the first or third aspects, and optionally one or more appropriate excipients or carriers.

In a fifth aspect, the invention provides the combination as defined in the first or third aspects, or the composition as defined in the fourth aspect, for use as a medicament.

### Detailed description of the invention

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

For purposes of the present invention, any ranges given include both the lower and the upper end-points of the range. Ranges given, such as concentrations and the like, should be considered approximate, unless specifically stated.

As used herein, the indefinite articles "a" and "an" are synonymous with "at least one" or "one or more." Unless indicated otherwise, definite articles used herein, such as "the" also include the plural of the noun.

As used herein, the term "about" refers to a range of values that is 15 % more or less than a fixed value.

As above disclosed, in a first aspect the present invention provides a combination comprising extracts of *Dittrichia viscosa, Syzygium aromaticum, Vaccinium myrtillus, Calendula officinalis, Euphrasia officinalis, Melilotus officinalis, Mentha spicata, Mimosa* tenuiflora and *Hordeum vulgare.*

As used herein, "extract" or "plant extract" refers to a concentrated preparation of one or various parts of a plant obtained by isolating the active constituents from the plant by suitable means. Depending on the physicochemical features of these active ingredients, suitable means for their isolation can be used, for example, aqueous solutions, organic solvents, microwave or supercritical fluids extraction. Plant extracts contain not only one but multiple constituents, many of them biologically active. Often, the beneficial effect is derived from the combination of many of these active compounds, even though in some cases there is one particular compound that is mainly responsible for most of the activity. The plant extracts, in particular the water plant extracts, of the combination of the first aspect can be prepared by conventional methods known to those skilled in the art.

In one particular embodiment of the first aspect, optionally in combination with any of the embodiments provided above or below, the *Dittrichia viscosa* extract is a leaf extract. In another particular embodiment, the *Syzygium aromaticum* extract is a flower bud extract. In an even more particular embodiment, the combination comprises a *Dittrichia viscosa* leaf extract and a *Syzygium aromaticum* flower bud extract, particularly, wherein the extracts are water extracts.

In one particular embodiment of the first aspect, optionally in combination with any of the embodiments provided above or below, the combination comprises *Dittrichia viscosa* leaf extract, *Syzygium aromaticum* flower bud extract, *Vaccinium myrtillus* leaf extract, and *Calendula officinalis* flower petal extract.

In an embodiment of the first aspect, the combination comprises at least one of *Dittrichia viscosa* leaf extract; *Syzygium aromaticum* flower bud extract; *Vaccinium myrtillus* leaf extract; *Calendula officinalis* flower extract; *Euphrasia officinalis* leaf and/or flower extract; *Melilotus officinalis* leaf and/or flower extract; *Mentha spicata* leaf extract; *Mimosa tenuiflora* leaf extract; and *Hordeum vulgare* seed extract.

In another embodiment, the combination comprises *Dittrichia viscosa* leaf extract, *Syzygium aromaticum* flower bud extract, *Vaccinium myrtillus* leaf extract, *Calendula officinalis* flower extract, *Euphrasia officinalis* leaf and/or flower extract, *Melilotus officinalis* leaf and/or flower extract, *Mentha spicata* leaf extract, *Mimosa tenuiflora* leaf extract and *Hordeum vulgare* seed extract.

In another embodiment of the first aspect, optionally in combination with any of the embodiments provided above or below, the combination comprises an extract selected from the group consisting of *Vaccinium myrtillus* leaf extract, *Calendula officinalis* flower petal extract, *Euphrasia officinalis* leaf and flower extract, *Melilotus officinalis* leaf and flower extract, *Mentha spicata* leaf extract, *Mimosa tenuiflora* leaf extract, *Hordeum vulgare* seed extract, and combinations thereof.

In one particular embodiment of the first aspect, optionally in combination with any of the embodiments provided above or below, the combination comprises or consists of *Dittrichia viscosa* leaf extract, *Syzygium aromaticum* flower bud extract, *Vaccinium myrtillus* leaf extract, *Calendula officinalis* flower petal extract, *Euphrasia officinalis* leaf and flower extract, *Melilotus officinalis* leaf and flower extract, *Mentha spicata* leaf extract, *Mimosa tenuiflora* leaf extract, and *Hordeum vulgare* seed extract.

In one particular embodiment of the first aspect, the extracts are water extracts. This embodiment is particularly disclosed in combination with any of the embodiments provided above regarding the extracts of the combination.

As above disclosed, the invention provides in a second aspect a process for the production of the combination as defined in the first aspect.

The process of the second aspect can be performed by boiling the dry material of all plants together in the same pot or by boiling the dry plant material of each plant separately in different pots. In case that they are boiled separately, either the plurality of mixtures obtained after boiling, or the plurality of extracts obtained after distilling, have to be combined in order to obtain the combination as defined in the first aspect. When the dry plant material is boiled separately in water, it is meant to encompass that the dry plant material of each plant is boiled separately, or that the dry plant material of various plants are boiled in groups.

As used herein, "boiling the dry plant material in water" refers to the process of heating the water that contains the dry plant material at least until it reaches its boiling point. The heating may be stopped right after the boiling point is reached or it can continue for a period of time; preferably, it is stopped right after the boiling point is reached.

In one particular embodiment of the second aspect, optionally in combination with any of the embodiments provided above or below, the process comprises the steps of (a) providing dry plant material of *Dittrichia viscosa, Syzygium aromaticum, Vaccinium myrtillus,* Calendula officinalis, *Euphrasia officinalis, Melilotus officinalis, Mentha spicata, Mimosa* tenuiflora and *Hordeum vulgare;* (b) boiling the dry plant material of (a) together in water, thereby obtaining a mixture; and (c) distilling the mixture of (b) so as to obtain the combination as defined in the first aspect.

In one particular embodiment of the second aspect, optionally in combination with any of the embodiments provided above or below, the process comprises the steps of (a) providing dry plant material of *Dittrichia viscosa, Syzygium aromaticum, Vaccinium myrtillus,* Calendula officinalis, *Euphrasia officinalis, Melilotus officinalis, Mentha spicata, Mimosa* tenuiflora and *Hordeum vulgare;* (b) boiling the dry plant material of (a) separately in water, thereby obtaining a plurality of mixtures; (b') distilling the plurality of mixtures of (b) thereby obtaining a plurality of extracts; and (c') combining the plurality of extracts of (b') so as to obtain the combination as defined in the first aspect.

In one particular embodiment of the second aspect, optionally in combination with any of the embodiments provided above or below, the process comprises the steps of (a) providing dry plant material of *Dittrichia viscosa* and *Syzygium aromaticum, Vaccinium myrtillus,* Calendula officinalis, *Euphrasia officinalis, Melilotus officinalis, Mentha spicata, Mimosa* tenuiflora and *Hordeum vulgare;* (b') boiling the dry plant material of (a) separately in water, thereby obtaining a plurality of mixtures; (b") combining the plurality of mixtures of (b'), thereby obtaining a single mixture; and (c) distilling the single mixture of (b") so as to obtain the combination as defined in the first aspect.

In one particular embodiment of the second aspect, optionally in combination with any of the embodiments provided above or below, the dry plant material of *Dittrichia viscosa* and *Syzygium aromaticum* is provided at a weight ratio (weight/weight) from 1.5:0.2 to 1.5:5; from 1.5:0.5 to 1.5:3; or from 1:5:0.8 to 1:5:2, more particularly, at a weight ratio of about 1.5:1.

The term "weight ratio" refers to the relation of weights of the dry plant materials indicated.

For instance, a weight ratio (weight/weight) of 1.5:1 between the *Dittrichia viscosa* and *Syzygium aromaticum* dry plant materials refers to 1.5 units of *Dittrichia viscosa* dry plant material for every 1 unit of *Syzygium aromaticum* plant material. In the present invention the plant material used to prepare the extracts is dry plant material, therefore the weight is the dry weight.

In one particular embodiment of the second aspect, optionally in combination with any of the embodiments provided above or below, the dry plant material provided in step (a) comprises or consists of *Dittrichia viscosa* leaves, *Syzygium aromaticum* flower buds, *Vaccinium myrtillus* leaves, *Calendula officinalis* flower petals, *Euphrasia officinalis* leaves and flowers, *Melilotus officinalis* leaves and flowers, *Mentha spicata* leaves, *Mimosa tenuiflora* leaves, and *Hordeum vulgare* seeds.

In one particular embodiment of the second aspect, optionally in combination with any of the embodiments provided above or below, the dry plant material of *Dittrichia viscosa, Syzygium aromaticum, Vaccinium myrtillus,* and *Calendula officinalis,* is provided at a weight ratio from 1.5:0.2:0.3:0.4 to 1.5:5:7.5:10; or from 1.5:0.5:0.7:1 to 1.5:2:2.5:3; particularly, at a weight ratio of about 1.5:1:1.5:2.

In one particular embodiment of the second aspect, optionally in combination with any of the embodiments provided above or below, the dry plant material of *Dittrichia viscosa, Syzygium aromaticum, Vaccinium myrtillus, Euphrasia officinalis, Melilotus officinalis, Mentha spicata, Mimosa Tenuiflora, Hordeum vulgare* is provided at a weight ratio from 1.5:0.2:0.3:0.4:0.4:0.4:0.2:0.3:0.4 to 1.5:5:7.5:10:10:10:5:7.5:10; or from 1.5:0.5:0.7:1:1:1:0.5:0.7:1 to 1.5:2:2.5:3:3:3:2:5:3; particularly, at a weight ratio of about 1.5:1:1.5:2:2:2:1:1.5:2.

In one particular embodiment of the second aspect, optionally in combination with any of the embodiments provided above or below, the dry plant material of *Dittrichia viscosa* is provided at a concentration from 0.05 % w/v to 5 % w/v; from 0.1 % w/v to 3 % w/v; or from 0.3 % w/v to 2 % w/v; particularly, at about 0.5 % w/v.

As used herein, "% weight/volume " or "% w/v" of a component refers to the amount of the single component relative to the total volume of the boiling reaction. In the present invention, the % w/v of a dry plant material refers to the dry weight or mass of the dry plant material relative to the total volume of water in which the dry plant material is boiled.

In one particular embodiment of the second aspect, optionally in combination with any of the embodiments provided above or below, the dry plant material of *Syzygium aromaticum* is provided at a concentration from 0.03 % w/v to 3.3 % w/v; from 0.1 % w/v to 2 % w/v; or from 0.2 % w/v to 1 % w/v; particularly, at a concentration of about 0.33 % w/v.

In one particular embodiment of the second aspect, optionally in combination with any of the embodiments provided above or below, the dry plant material provided in step (a) comprises *Dittrichia viscosa* leaves and *Syzygium aromaticum* flower buds at a weight ratio from 1.5:0.2 to 1.5:5; from 1.5:0.5 to 1.5:3; from 1:5:0.8 to 1:5:2; or of about 1.5:1. In an even more particular embodiment, the dry plant material comprises *Dittrichia viscosa* leaves, *Syzygium aromaticum* flower buds, *Vaccinium myrtillus* leaves, and *Calendula officinalis* flower petals at a weight ratio from 1.5:0.2:0.3:0.4 to 1.5:5:7.5:10; from 1.5:0.5:0.7:1 to 1.5:2:2.5:3; or of about 1.5:1:1.5:2. In an even more particular embodiment, the dry plant material comprises or consists of *Dittrichia viscosa* leaves, *Syzygium aromaticum* flower buds, *Vaccinium myrtillus* leaves, *Calendula officinalis* flower petals, *Euphrasia officinalis* leaves and flowers, *Melilotus officinalis* leaves and flowers, *Mentha spicata* leaves, *Mimosa tenuiflora* leaves, and *Hordeum vulgare* seeds, at a weight ratio from 1.5:0.2:0.3:0.4:0.4:0.4:0.2:0.3:0.4 to 1.5:5:7.5:10:10:10:5:7.5:10; from 1.5:0.5:0.7:1:1:1:0.5:0.7:1 to 1.5:2:2.5:3:3:3:2:5:3; or of about 1.5:1:1.5:2:2:2:1:1.5:2.

In one particular embodiment of the second aspect, optionally in combination with any of the embodiments provided above or below, the dry plant material provided in step (a) comprises from 1 g to 10 g, from 2 g to 8 g, from 3 g to 7 g, from 4 g to 6 g, or of about 5 g of *Dittrichia viscosa* dry plant material per liter of water used for boiling in step (b).

In one particular embodiment of the second aspect, optionally in combination with any of the embodiments provided above or below, the step (b) is performed for a period of time from 1 s to 15 min, from 5 s to 10 min, from 30 s to 5 min, or from 1 min to 2 min. In a more particular embodiment, the step (b) is performed for at least 1 s, at least 5 s, at least 30 s, at least 1 min or at least 5 min. In a more particular embodiment, the step (b) is performed for more than 1 s, more than 5 s, more than 30 s, more than 1 min or more than 5 min.

In one particular embodiment of the second aspect, optionally in combination with any of the embodiments provided above or below, the process further comprises after step (b) and before step (c) the step of filtering and discarding the boiled dry plant material from the mixture.

In one particular embodiment of the second aspect, optionally in combination with any of the embodiments provided above or below, the distillation of step (c) is performed by simple distillation.

The term "simple distillation", as used herein, refers to a method for the selective boiling and subsequent condensation of one ore more components in a liquid mixture.

In one particular embodiment of the second aspect, optionally in combination with any of the embodiments provided above or below, the distillation of step (c) is performed at a temperature from 80 °C to 120 °C, from 90 °C to 110 °C, or about 100 °C.

In one particular embodiment of the second aspect, optionally in combination with any of the embodiments provided above or below, the distillation of step (c) is performed by simple distillation with a distillation yield from 20 % to 60 %, from 30 % to 50 %, or of about 40 %.

As above disclosed, in a third aspect the invention provides a combination as defined in the first aspect obtainable by the process as defined in the second aspect.

The combination "obtainable by" the process as defined above is used herein to define the combination by its preparation process and relates to the combination obtainable by the product process which comprises the steps a), b), and c) described above. For the purposes of the invention, the expressions "obtainable", "obtained" and equivalent expressions are interchangeably used, and in any case the expression "obtainable" includes the expression "obtained".

All the embodiments disclosed above regarding the combination of the first aspect of the invention are also meant to apply to the combination of the third aspect of the invention.

In a fourth aspect, the invention provides a composition comprising the combination of the first or third aspects.

In one particular embodiment of the fourth aspect, optionally in combination with any of the embodiments provided above or below, the composition is a pharmaceutical composition comprising a therapeutically effective amount of the combination as defined in any of the first or third aspects and optionally, one or more pharmaceutically acceptable excipients and/or carriers.

The expression "therapeutically effective amount" as used herein, refers to the amount of the combination that, when administered, is sufficient to prevent development of, or alleviate to some extent, one or more of the symptoms of the disease which is addressed (e.g., presbyopia). The particular dose of the combination administered according to this invention will of course be determined by the particular circumstances surrounding the case, including the compound administered, the route of administration, the particular condition being treated, and the similar considerations.

The expression "pharmaceutically acceptable excipients and/or carriers" refers to pharmaceutically acceptable materials, compositions or vehicles. Each component must be pharmaceutically acceptable in the sense of being compatible with the other ingredients of the pharmaceutical composition. It must also be suitable for use in contact with the tissue or organ of humans and animals without excessive toxicity, irritation, allergic response, immunogenicity or other problems or complications commensurate with a reasonable benefit/risk ratio.

Examples of suitable pharmaceutically acceptable excipients are solvents, dispersion media, diluents, or other liquid vehicles, dispersion or suspension aids, surface active agents, isotonic agents, thickening or emulsifying agents, preservatives, solid binders, lubricants and the like. Except insofar as any conventional excipient medium is incompatible with a substance or its derivatives, such as by producing any undesirable biological effect or otherwise interacting in a deleterious manner with any other component(s) of the pharmaceutical composition, its use is contemplated to be within the scope of this invention.

The relative amounts of the active ingredient, the pharmaceutically acceptable excipient, and/or any additional ingredients in a pharmaceutical composition of the invention will vary, depending upon the identity, size, and/or condition of the subject treated and further depending upon the route by which the composition is to be administered.

Pharmaceutically acceptable excipients used in the manufacture of pharmaceutical compositions include, but are not limited to, inert diluents, dispersing and/or granulating agents, surface active agents and/or emulsifiers, disintegrating agents, binding agents, preservatives, buffering agents, lubricating agents, and/or oils. Excipients such as colouring agents, coating agents, sweetening, and flavouring agents can be present in the composition, according to the judgment of the formulator.

Exemplary diluents include, but are not limited to, calcium carbonate, sodium carbonate, calcium phosphate, dicalcium phosphate, calcium sulfate, calcium hydrogen phosphate, sodium phosphate lactose, sucrose, cellulose, microcrystalline cellulose, kaolin, mannitol, sorbitol, inositol, sodium chloride, dry starch, corn-starch, powdered sugar, and combinations thereof.

Exemplary granulating and/or dispersing agents include, but are not limited to, potato starch, corn starch, tapioca starch, sodium starch glycolate, clays, alginic acid, guar gum, citrus pulp, agar, bentonite, cellulose and wood products, natural sponge, cation-exchange resins, calcium carbonate, silicates, sodium carbonate, cross-linked polyvinylpyrrolidone) (crospovidone), sodium carboxymethyl starch (sodium starch glycolate), carboxymethyl cellulose, cross-linked sodium carboxymethyl cellulose (croscarmellose), methylcellulose, pregelatinized starch (starch 1500), microcrystalline starch, water insoluble starch, calcium carboxymethyl cellulose, magnesium aluminum silicate (Veegum), sodium lauryl sulfate, quaternary ammonium compounds, and combinations thereof.

Exemplary binding agents include, but are not limited to, starch (e.g., corn-starch and starch paste); gelatin; sugars (e.g., sucrose, glucose, dextrose, dextrin, molasses, lactose, lactitol, mannitol); natural and synthetic gums (e.g., acacia, sodium alginate, extract of Irish moss, panwar gum, ghatti gum, mucilage of isapol husks, carboxymethylcellulose, methylcellulose, ethylcellulose, hydroxyethylcellulose, hydroxypropyl cellulose, hydroxypropyl methylcellulose, microcrystalline cellulose, cellulose acetate, polyvinylpyrrolidone), magnesium aluminium silicate (Veegum), and larch arabogalactan); alginates; polyethylene oxide; polyethylene glycol; inorganic calcium salts; silicic acid; polymethacrylates; waxes; water; alcohol; and combinations thereof.

Exemplary preservatives may include antioxidants, chelating agents, antimicrobial preservatives, antifungal preservatives, alcohol preservatives, acidic preservatives, and other preservatives. Exemplary antioxidants include, but are not limited to, alpha tocopherol, ascorbic acid, ascorbyl palmitate, ascorbyl stearate, ascorbyl oleate, butylated hydroxyanisole, butylated hydroxytoluene, monothioglycerol, potassium metabisulfite, propionic acid, propyl gallate, sodium ascorbate, sodium bisulfite, sodium metabisulfite, and sodium sulfite. Exemplary chelating agents include ethylenediaminetetraacetic acid (EDTA), citric acid monohydrate, disodium edetate, dipotassium edetate, edetic acid, fumaric acid, malic acid, phosphoric acid, sodium edetate, tartaric acid, and trisodium edetate.

Exemplary buffering agents include, but are not limited to, citrate buffer solutions, acetate buffer solutions, phosphate buffer solutions, ammonium chloride, calcium carbonate, calcium chloride, calcium citrate, calcium glubionate, calcium gluceptate, calcium gluconate, D-gluconic acid, calcium glycerophosphate, calcium lactate, propanoic acid, calcium levulinate, pentanoic acid, dibasic calcium phosphate, phosphoric acid, tribasic calcium phosphate, calcium hydroxide phosphate, potassium acetate, potassium chloride, potassium gluconate, potassium mixtures, dibasic potassium phosphate, monobasic potassium phosphate, potassium phosphate mixtures, sodium acetate, sodium bicarbonate, sodium chloride, sodium citrate, sodium lactate, dibasic sodium phosphate, monobasic sodium phosphate, sodium phosphate mixtures, tromethamine, magnesium hydroxide, aluminum hydroxide, alginic acid, pyrogen-free water, isotonic saline, Ringer's solution, ethyl alcohol, and combinations thereof.

Exemplary lubricating agents include, but are not limited to, magnesium stearate, calcium stearate, stearic acid, silica, talc, malt, glyceryl behanate, hydrogenated vegetable oils, polyethylene glycol, sodium benzoate, sodium acetate, sodium chloride, leucine, magnesium lauryl sulfate, sodium lauryl sulfate, and combinations thereof.

In one embodiment of the fourth aspect, optionally in combination with any of the embodiments provided above or below, the pharmaceutical composition is an ophthalmic pharmaceutical composition, optionally comprising one or more ophthalmic acceptable excipients and/or carriers.

It is understood by the skilled in the art that when the pharmaceutical composition is an ophthalmic pharmaceutical composition the pharmaceutically acceptable excipients and/or carriers must be ophthalmically acceptable excipients and/or carriers; that is, suitable specifically for contact with the tissues of the eye, and the area surrounding the eye without excessive toxicity, irritation, allergic response, or other problem complications commensurate with a reasonable benefit/risk ratio. In a more particular embodiment, the pharmaceutical composition is an ophthalmic pharmaceutical composition for topical administration into the eye surface. Topical ocular dosage forms for use according to the invention are those appropriated for allowing the active ingredients to be stable and to be applied topically onto the surface of eye or eyelid.

With the aim of making such compositions easy to be administered by the patient, and also to avoid disturbance of vision as much as possible when the compositions are applied, the compositions are preferably in the form of eye drop solutions, more in particular eye drop transparent solutions.

Thus, in a particular embodiment of the fourth aspect, optionally in combination with any of the embodiments provided above or below, the ophthalmic pharmaceutical composition is in the form of eye drop, eye mist, eye ointment, or eye gel. In a more particular embodiment, it is in the form of eye drop. In a more particular embodiment, the form of eye drop is sterilized and, optionally, contained in disposable single-dose eye drop containers for ophthalmic use. This form is advantageous because these compositions do not require the inclusion of preservatives in the composition. The term "sterile" refers to a composition that has been aseptically processed and that is devoid of viable bacteria, fungi or other microorganisms.

The ophthalmic pharmaceutical compositions of the present invention can be prepared according to methods well known in the state of the art. The appropriate excipients and/or carriers, as well as any pH adjusting agents, and their amounts, can readily be determined by those skilled in the art according to the type of formulation being prepared.

As above described, in a fifth aspect the invention provides the combination of the first or third aspects or the composition of the fourth aspect for use a medicament.

In a particular embodiment of the fifth aspect, optionally in combination with any of the embodiments provided above or below, the combination as defined in the first or third aspects, or the composition as defined in the fourth aspect, are for use in the treatment and/or prevention of an ocular disease. In a more particular embodiment, the ocular disease in presbyopia.

This embodiment can also be formulated as the use of the combination or the composition of the invention for the manufacture of a medicament for the prevention and/or treatment of an ocular disease, in particular, presbyopia. This aspect can also be formulated as a method for preventing and/or treating an ocular disease, in particular presbyopia, the method comprising administering a therapeutically effective amount of the combination or the composition of the invention, together with pharmacologically acceptable excipients and/or carriers to a subject in need thereof.

In a particular embodiment of the fifth aspect, optionally in combination with any of the embodiments provided above or below, the prevention and/or treatment of presbyopia comprises the topical administration into the eye of the combination or the composition of the invention.

In a particular embodiment of the fifth aspect, optionally in combination with any of the embodiments provided above or below, the prevention and/or treatment of presbyopia comprises administering the combination of the first or third aspect, or the composition of the fourth aspect, once daily into each eye. More particularly, the prevention and/or treatment of presbyopia comprises administering one drop of the combination or the composition daily into each eye. Even more particularly, the prevention and/or treatment of presbyopia comprises administering from 0.01 ml to 0.1 ml, from 0.04 ml to 0.06 ml, or about 0.05 ml, of the combination or the composition daily into each eye.

In a particular embodiment of the fifth aspect, optionally in combination with any of the embodiments provided above or below, the prevention and/or treatment of presbyopia comprises administering the combination of the first or third aspect, or the composition of the fourth aspect, once daily into each eye for at least 7 days, at least 15 days, at least 25 days, at least 35 days, or at least 45 days; in particular, for about 45 days.

In another particular embodiment of the sixth aspect, optionally in combination with any of the embodiments provided above or below, the pharmaceutical compositions for use as disclosed above is formulated for administration to a subject once or twice per eye every 0.25, 0.5, 1, 2, 4, 6, 8, 12, 16, 24 and 48 hours. In a more particular embodiment, is formulated for administration to a subject once or twice per eye every 24 hours.

In a particular embodiment of the fifth aspect, optionally in combination with any of the embodiments provided above or below, the prevention and/or treatment of presbyopia comprises (a) administering a pharmaceutical composition comprising a therapeutically effective amount of a *Dittrichia viscosa* extract; a therapeutically effective amount of a *Syzygium aromaticum* extract; a therapeutically effective amount of an extract of *Vaccinium myrtillus, Calendula officinalis, Euphrasia officinalis, Melilotus officinalis, Mentha spicata, Mimosa tenuiflora,* and *Hordeum vulgare;* and optionally, one or more pharmaceutically acceptable excipients and/or carriers; or alternatively, (b) administering simultaneously, sequentially, or separately (b1) a pharmaceutical composition comprising a therapeutically effective amount of a *Dittrichia viscosa* extract; and optionally, one or more pharmaceutically acceptable excipients and/or carriers; (b2) a pharmaceutical composition comprising a therapeutically effective amount of a *Syzygium aromaticum* extract; and optionally, one or more pharmaceutically acceptable excipients and/or carriers; and (b3) a pharmaceutical composition comprising a therapeutically effective amount an extract from *Vaccinium myrtillus, Calendula officinalis, Euphrasia officinalis, Melilotus officinalis, Mentha spicata, Mimosa tenuiflora,* and *Hordeum vulgare;* and optionally, one or more pharmaceutically acceptable excipients and/or carriers. The therapeutically effective amount of the extracts from *Vaccinium myrtillus, Calendula officinalis, Euphrasia officinalis, Melilotus officinalis, Mentha spicata, Mimosa tenuiflora, Hordeum vulgare* can be also provided in a single pharmaceutical compositions or in various independent pharmaceutical compositions.

In a particular embodiment of the fifth aspect, the prevention and/or treatment of presbyopia comprises (a) administering a pharmaceutical composition comprising: a therapeutically effective amount of extracts from *Dittrichia viscosa, Syzygium aromaticum, Vaccinium myrtillus, Calendula officinalis, Euphrasia officinalis, Melilotus officinalis, Mentha spicata, Mimosa tenuiflora,* and *Hordeum vulgare;* and optionally, one or more pharmaceutically acceptable excipients and/or carriers; or alternatively, (b) administering simultaneously, sequentially, or separately: (b1) a pharmaceutical composition comprising a therapeutically effective amount of a *Dittrichia viscosa* extract; and optionally, one or more pharmaceutically acceptable excipients and/or carriers; (b2) a pharmaceutical composition comprising a therapeutically effective amount of a *Syzygium aromaticum* extract; and optionally, one or more pharmaceutically acceptable excipients and/or carriers; (b3) a pharmaceutical composition comprising a therapeutically effective amount of a *Vaccinium myrtillus* extract; and optionally, one or more pharmaceutically acceptable excipients and/or carriers; (b4) a pharmaceutical composition comprising a therapeutically effective amount of a *Calendula officinalis* extract; and optionally, one or more pharmaceutically acceptable excipients and/or carriers; (b5) a pharmaceutical composition comprising a therapeutically effective amount of a *Euphrasia officinalis* extract; and optionally, one or more pharmaceutically acceptable excipients and/or carriers; (b6) a pharmaceutical composition comprising a therapeutically effective amount of a *Melilotus officinalis* extract; and optionally, one or more pharmaceutically acceptable excipients and/or carriers; (b7) a pharmaceutical composition comprising a therapeutically effective amount of a *Mentha spicata* extract; and optionally, one or more pharmaceutically acceptable excipients and/or carriers; (b8) a pharmaceutical composition comprising a therapeutically effective amount of a *Mimosa tenuiflora* extract; and optionally, one or more pharmaceutically acceptable excipients and/or carriers; and (b9) a pharmaceutical composition comprising a therapeutically effective amount of a *Hordeum vulgare* extract; and optionally, one or more pharmaceutically acceptable excipients and/or carriers.

The present inventors found that when the administration of the combination of the invention is followed by visual accommodation training, its therapeutic effect is enhanced.

Thus, in a particular embodiment of the fifth aspect, optionally in combination with any of the embodiments provided above or below, the prevention and/or treatment of presbyopia further comprises performing visual accommodation training after the administration of the combination or the composition of the invention; in particular, within about 30 minutes after the administration. In an even more particular embodiment, the visual accommodation training is performed for about 15 minutes. As used herein, "visual accommodation training" refers to visual exercises that activate the ciliary muscles by alternately repeating negative and positive accommodation.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples are provided by way of illustration, and they are not intended to be limiting of the present invention. Furthermore, the present invention covers all possible combinations of particular and preferred embodiments described herein.

### Examples

### Example 1 - "Monaguina" extract preparation

The following dry plant parts were used for the preparation of the combination "Monaguina":

**Table 1**

| **Plant species** | **Amount (g) per liter** | **Plant part** |
|---|---|---|
| *Dittrichia viscosa* | 5 | Leaves |
| *Syzygium aromaticum* | 3,33 | Flower buds |
| *Vaccinium myrtillus* | 5 | Leaves |
| *Calendula officinalis* | 10 | Flower petals |
| *Euphrasia officinalis* | 10 | Leaves and flowers |
| *Melilotus officinalis* | 10 | Leaves and flowers |
| *Mentha spicata* | 3,33 | Leaves |
| *Mimosa Tenuiflora* | 5 | Leaves |
| *Hordeum vulgare* | 10 | Seeds |

*Dittrichia viscosa* and *Syzygium aromaticum* were found to be the essential plants that provide the extracts with the constitutes necessary for their therapeutic use. *Vaccinium myrtillus* and *Calendula officinalis* were added to the mixture to further increase the therapeutic efficacy of the extract. The materials from the other plants (*Euphrasia officinalis, Melilotus officinalis, Mentha spicata, Mimosa Tenuiflora,* and *Hordeum vulgare*) were added to increase the stability of the final formulation.

The dry plant parts of Table 1 were added to three liters of distilled water in an aseptic vessel. The mixture was heated until reaching the boiling point and then it was left to cool at room temperature. The mixture was filtered and distilled by simple distillation with a distillation yield of around 40 % for the obtention of the final extract.

### Example 2 - Treatment protocol

A pilot clinical trial was carried out to evaluate the efficacy and tolerability of the combination of the invention for the treatment of presbyopia. The clinical trial was single-blind, randomized, multicenter, parallel, and placebo-controlled.

### Treatment scheme

Each subject included in the study was administered during seven consecutive days with two drops (one in each eye) of the extract described in Example 1 or the control product according to a randomization table, followed by a session of accommodation visual training for 15 minutes.

### Randomization

The assignment of each subject to one of the two preparations evaluated in the study was randomized using a randomization table previously generated by computer, in which it was controlled that half of the subjects were assigned to the experimental preparation and the other half to the control preparation.

### Inclusion Criteria

Subjects met all of the following criteria:
1. Subjects of both sexes, between 40 and 55 years old 2.
2. Normal binocular vision
3. High motivation to receive treatment (score above 75% on the VAS).
4. Not having participated in another clinical trial during the two months preceding the start of the current study.
5. Free acceptance to participate in the trial, with written informed consent to the current study.

### Exclusion Criteria

Subjects presenting one or more of the following criteria were NOT eligible to participate in this study:
1. Prior history of alcohol or drug dependence.
2. Habitual users of other types of drugs such as antihistamines, corticosteroids, anxiolytics and antidepressants, and psychotropic drugs in general.
3. History of allergy, idiosyncrasy or hypersensitivity to drugs.
4. Pregnancy or lactation.
5. Positive serology for hepatitis B, C or HIV.
6. History or clinical evidence of hepatic or cardiovascular pathology, or patients with hypokalemia, hyperestrogenism or diabetes mellitus.
7. History or clinical evidence of ocular pathologies such as conjunctival and corneal inflammation and infections, dystrophies, degenerations, ectasias and in general, pathologies of the posterior pole.
8. Strabismic and organic refractive amblyopia.
9. Strabismus of any type
10. Having undergone surgery during the previous 6 months.

### Number of subjects

Given the exploratory nature of the study (pilot trial), the sample size was not formally calculated, and a sample size of 12 subjects was considered adequate.

### Pre-study evaluation. Selection of subjects

The subjects were examined prior to the beginning of the study, in order to collect (in some cases update) the data listed below, which was recorded in the corresponding individual monitoring sheet for each volunteer.
- Personal data.
- Anamnesis: personal history: detailed medical history, pharmacological and surgical history.
- Degree of motivation and confidence to stop wearing near glasses (inclusion criterion: score over 75% in the VAS).
- Usual optometric treatment (glasses, contact lenses, prisms, vision therapy, filters...)
- Uncorrected near VA (distance 40 cm)
- Distance and near ocular refraction
- Accommodative status: accommodative amplitude and mono and binocular accommodative flexibility (study parameters).
- Binocular status: near convergence point occlusion, planar fusion and stereopsis tests.
- Ocular health: biomicroscopy, ophthalmoscopy and pupillary reflexes.

### Description of experimental period

Prior to the start of the experimental phase, a brief semi-structured interview was carried out to assess their general physical and ocular condition, recording any anomaly or cause for exclusion. Likewise, their motivation and confidence in the treatment was evaluated by means of subjective motivation scales. All subjects included in the study underwent a period of treatment lasting 7 consecutive days, committing themselves to perform the proposed accommodative visual therapy during the prescribed 15 minutes following the investigator's indications. 1 day after the end of the treatment, each subject went for optometric evaluation. The follow-up period was extended during the following 3 months, by means of a monthly optometric examination. The follow-up period had a duration of 3 months except in the case of abandonment or withdrawal. In the case of premature discontinuation and having received the artificial tear during the treatment period, the possibility of receiving the treatment with the eye drops WAS offered, following the same guidelines established in the study protocol. The results obtained in these cases were not included in the statistical analysis.

### Description of the evaluation procedure

Accommodative amplitude was quantified by the measurement of the near point of accommodation, which assesses the maximum focusing ability of the lens.

Accommodative flexibility was assessed by measuring in cycles per minute the ability of each subject to relax and stimulate accommodation at 40 cm. Two pairs of lenses of the same dioptric power but of opposite sign were used for the measurement, in this case, and taking into account the age of the subjects in the sample, they were +/- 1.50 D lenses.

The degree of motivation of the subject was assessed by means of two 100 mm visual analog scales (VAS/100) on which the subject had to mark with a line the position that best reflected his/her level of motivation to start and follow the treatment and the confidence he/she had to stop using glasses for near vision.

The scales were constructed in such a way that the maximum motivation is close to 100 and the minimum to zero.

### Treatment period

Prior to the start of the treatment, a record of the optometric parameters was made, including the values of the accommodative and binocular measurements and the ocular health status of each subject, as specified in point above. Likewise, the subjects had to assess their degree of motivation and confidence in the treatment by means of 100 mm visual analog scales (VAS), so that the maximum intensity of the state is close to 100 and the minimum to zero.

For the collection of adverse events, the investigator asked, "Do you have any symptoms or discomfort?" evaluating each of them according to the guidelines, any adverse event spontaneously referred by the subject was recorded.

### Follow-up period

After 7 days of starting the treatment, the subjects had to return to the center for the relevant optometric evaluations of uncorrected near VA and of the subject's accommodative status: accommodative amplitude and accommodative flexibility. A brief semi-structured interview was also be carried out in order to record possible adverse events. In addition, subjects had their state of motivation and confidence in the treatment assessed.

### Evaluation of the response

Main variable. The main variables of the study were the changes experienced in the accommodative state (accommodative amplitude and accommodative flexibility), 7 days after the beginning of the treatment.

Secondary Variables. The secondary efficacy variables were: The increase in uncorrected near VA. Changes in the scores obtained in the subjective evaluation scales of the degree of motivation and confidence in the treatment. (VAS /100)

### Example 3 - Results

The results obtained in the treatment protocol of Example 2 after 7 days of treatment are summarized in the following table:

**Table 2**

| **Patient** | **1** | **2** | **3** | **4** | **5** | **6** | **7** | **8** | **9** | **10** | **11** | **12** |
|---|---|---|---|---|---|---|---|---|---|---|---|---|
| **Age** | 46 | 45 | 44 | 48 | 42 | 44 | 47 | 50 | 43 | 47 | 42 | 45 |
| | | | | | | | | | | | | |

| **REFRACTIVE STATUS DISTANCE VISION:** | | | | | | | | | | | | |
|---|---|---|---|---|---|---|---|---|---|---|---|---|
| **RE.** | -0.25 | -1.00 | N | N | N | N | -0.25 | N | N | N | +1.0 0 | N |
| **LE.** | -0.75 | -1.50 | N | N | N | N | -0.25 | N | N | N | +1.2 5-0.50 *30° | N |
| | | | | | | | | | | | | |

| **OPTOMETRIC PARAMETRERS BEFORE TRETATMENT:** | | | | | | | | | | | | |
|---|---|---|---|---|---|---|---|---|---|---|---|---|
| **Near VA sc binocular** | 0.6 | 0.9 | 0.8 | 0.4 | 0.9 | 0.8 | 0.6 | 0.2 | 0.8 | 0.4 | 0.7 | 0.5 |
| **NPA binocular (cm)** | 35 | 15 | 33 | 45 | 30 | 35 | 29 | 50 | 30 | 42 | 36 | 35 |
| **AF binocular (cpm)** | 0 | 4 | 1 | 0 | 3 | 2 | 0 | 0 | 2 | 0 | 1 | 1 |
| | | | | | | | | | | | | |

| **OPTOMETRIC PARAMETRERS AFTER TRETATMENT:** | | | | | | | | | | | | |
|---|---|---|---|---|---|---|---|---|---|---|---|---|
| **Near VA sc binocular** | 0.8 | 1.0 | 1.0 | 0.6 | 1.0 | 1.0 | 0.8 | 0.4 | 1.0 | 0.6 | 0.9 | 0.8 dif |
| **NPA binocular (cm)** | 25 | 12 | 25 | 38 | 23 | 24 | 23 | 42 | 22 | 34 | 25 | 24 |
| **AF binocular (cpm)** | 2 | 10 | 3 | 1 | 6 | 4 | 2 | 0 | 3 | 1 | 3 | 2 |

RE stands for right eye; *LE* stands for left eye; *Near VA* so stands for near visual acuity without correction; *NPA* stands for near point of accommodation; *AF* stands for accommodative flexibility; and *N* stands for neutral

Patients 1 to 12 were administered with the combination of the invention. As shown in Table 2 above, all subjects treated with the combination of the invention showed improved near vision parameters -enhanced the near visual acuity, reduced near point of accommodation, and enhanced accommodative flexibility- after only 7 days of treatment. Notably, none of the subjects reported any side-effects associated to the administration of the combination. Patients administered with placebo (distilled water) did not show any improvement in near vision parameters.

Hence, these results show that the combination of the invention constitutes an efficient and safe treatment for presbyopia.

## Claims

1. A combination comprising extracts of *Dittrichia viscosa, Syzygium aromaticum, Vaccinium myrtillus, Calendula officinalis, Euphrasia officinalis, Melilotus officinalis, Mentha spicata, Mimosa* tenuiflora and *Hordeum vulgare.*

2. The combination according to claim 1, which comprises at least one of:
- *Dittrichia viscosa* leaf extract;
- *Syzygium aromaticum* flower bud extract;
- *Vaccinium myrtillus* leaf extract;
- *Calendula officinalis* flower extract;
- *Euphrasia officinalis* leaf and/or flower extract;
- *Melilotus officinalis* leaf and/or flower extract;
- *Mentha spicata* leaf extract;
- *Mimosa tenuiflora* leaf extract; and
- *Hordeum vulgare* seed extract.

3. The combination according to any of claims 1-2, which comprises *Dittrichia viscosa* leaf extract, *Syzygium aromaticum* flower bud extract, *Vaccinium myrtillus* leaf extract, *Calendula officinalis* flower extract, *Euphrasia officinalis* leaf and/or flower extract, *Melilotus officinalis* leaf and/or flower extract, *Mentha spicata* leaf extract, *Mimosa tenuiflora* leaf extract and *Hordeum vulgare* seed extract.

4. The combination according to any of claims 1-3, wherein the extracts are water extracts.

5. A process for the production of the combination as defined in any of claims 1-4, the process comprising the steps of:
(a) providing dry plant material of *Dittrichia viscosa, Syzygium aromaticum, Vaccinium myrtillus, Calendula officinalis, Euphrasia officinalis, Melilotus officinalis, Mentha spicata, Mimosa* Tenuiflora and *Hordeum vulgare;*
(b) boiling the dry plant material of (a) together in water, thereby obtaining a mixture; and
(c) distilling the mixture of (b) so as to obtain the combination as defined in any of claims 1-4;
or, alternatively,
(a) providing dry plant material of *Dittrichia viscosa, Syzygium aromaticum, Vaccinium myrtillus, Calendula officinalis, Euphrasia officinalis, Melilotus officinalis, Mentha spicata, Mimosa* Tenuiflora and *Hordeum vulgare;*
(b) boiling the dry plant material of (a) separately in water, thereby obtaining a plurality of mixtures;
(c) distilling the plurality of mixtures of (b) thereby obtaining a plurality of extracts; and
(c') combining the plurality of extracts of (c) so as to obtain the combination as defined in any of claims 1-4;
or, alternatively;
(a) providing dry plant material of *Dittrichia viscosa, Syzygium aromaticum, Vaccinium myrtillus, Calendula officinalis, Euphrasia officinalis, Melilotus officinalis, Mentha spicata, Mimosa* Tenuiflora and *Hordeum vulgare;*
(b) boiling the dry plant material of (a) separately in water, thereby obtaining a plurality of mixtures;
(b') combining the plurality of mixtures of (b), thereby obtaining a single mixture; and
(c) distilling the single mixture of (b') so as to obtain the combination as defined in any of claims 1-4.

6. The process according to claim 5, wherein the dry plant material of *Dittrichia viscosa* and *Syzygium aromaticum* are provided in step (a) at a weight ratio (weight/weight) from 1.5:0.2 to 1.5:5; particularly, at a weight ratio of about 1.5:1.

7. The process according to any of claims 5-6, wherein dry plant material of *Dittrichia viscosa, Syzygium aromaticum, Vaccinium myrtillus, Calendula officinalis, Euphrasia officinalis, Melilotus officinalis, Mentha spicata, Mimosa Tenuiflora, Hordeum vulgare* are provided in step (a) at a weight ratio from 1.5:0.2:0.3:0.4:0.4:0.4:0.2:0.3:0.4 to 1.5:5:7.5:10:10:10:5:7.5:10; particularly, at a weight ratio of about 1.5:1:1.5:2:2:2:1:1.5:2.

8. A combination as defined in any of claims 1-4 which is obtainable by the process as defined in any of claims 5-7.

9. A composition comprising the combination as defined in any of claims 1-4 or 8, and optionally one or more appropriate excipients or carriers.

10. The composition according to claim 9 which is a pharmaceutical composition comprising a therapeutically effective amount of the combination as defined in any of claims 1-4 or 8, and optionally one or more pharmaceutically acceptable excipients and/or carriers;

11. The composition according to any of claims 9-10, which is an ophthalmic pharmaceutical composition, optionally comprising one or more ophthalmic acceptable excipients and/or carriers.

12. The combination as defined in claims 1-4 or 8, or the composition as defined in any of claims 9-11 for use as a medicament.

13. The combination or the composition for use according to claim 12, which is for use in the treatment and/or prevention of presbyopia.

14. The combination or the compositions for use according to claim 13, wherein the treatment and/or prevention of presbyopia comprises administering the combination or the composition once daily into each eye.

15. The combination or the compositions for use according to any of claims 13-14, wherein the prevention and/or treatment of presbyopia comprises:
(a) administering a pharmaceutical composition comprising:
a therapeutically effective amount of extracts from *Dittrichia viscosa, Syzygium aromaticum, Vaccinium myrtillus, Calendula officinalis, Euphrasia officinalis, Melilotus officinalis, Mentha spicata, Mimosa* tenuiflora and *Hordeum vulgare;* and
optionally, one or more pharmaceutically acceptable excipients and/or carriers; or alternatively,
(b) administering simultaneously, sequentially, or separately:
(b1) a pharmaceutical composition comprising a therapeutically effective amount of a *Dittrichia viscosa* extract; and optionally, one or more pharmaceutically acceptable excipients and/or carriers;
(b2) a pharmaceutical composition comprising a therapeutically effective amount of a *Syzygium aromaticum* extract; and optionally, one or more pharmaceutically acceptable excipients and/or carriers;
(b3) a pharmaceutical composition comprising a therapeutically effective amount of a *Vaccinium myrtillus* extract; and optionally, one or more pharmaceutically acceptable excipients and/or carriers;
(b4) a pharmaceutical composition comprising a therapeutically effective amount of a *Calendula officinalis* extract; and optionally, one or more pharmaceutically acceptable excipients and/or carriers;
(b5) a pharmaceutical composition comprising a therapeutically effective amount of a *Euphrasia officinalis* extract; and optionally, one or more pharmaceutically acceptable excipients and/or carriers;
(b6) a pharmaceutical composition comprising a therapeutically effective amount of a *Melilotus officinalis* extract; and optionally, one or more pharmaceutically acceptable excipients and/or carriers;
(b7) a pharmaceutical composition comprising a therapeutically effective amount of a *Mentha spicata* extract; and optionally, one or more pharmaceutically acceptable excipients and/or carriers;
(b8) a pharmaceutical composition comprising a therapeutically effective amount of a *Mimosa tenuiflora* extract; and optionally, one or more pharmaceutically acceptable excipients and/or carriers; and
(b9) a pharmaceutical composition comprising a therapeutically effective amount of a *Hordeum vulgare* extract; and optionally, one or more pharmaceutically acceptable excipients and/or carriers.

## Patentansprüche

1. Eine Kombination umfassend Extrakte von *Diffrichia viscosa, Syzygium aromaticum, Vaccinium myrtillus, Calendula officinalis, Euphrasia officinalis, Melilotus officinalis, Mentha spicata, Mimosa tenuiflora,* und *Hordeum vulgare.*

2. Die Kombination nach Anspruch 1, welche mindestens eines von den Folgenden umfasst:
- Blattextrakt aus *Dittrichia viscosa;*
- Blütenknospenextrakt aus *Syzygium aromaticum;*
- Blattextrakt aus *Vaccinium myrtillus;*
- Blütenextrakt aus *Calendula officinalis;*
- Blatt- und/oder Blütenextrakt aus *Euphrasia officinalis;*
- Blatt- und/oder Blütenextrakt aus *Melilotus officinalis;*
- Blattextrakt aus *Mentha spicata;*
- Blattextrakt aus *Mimosa tenuiflora;* und
- Samenextrak aus *Hordeum vulgare.*

3. Die Kombination nach einem der Ansprüche 1 bis 2, welche Blattextrakt aus *Diffrichia* viscosa, Blütenknospenextrakt aus *Syzygium aromaticum,* Blattextrakt aus *Vaccinium myrtillus,* Blütenextrakt aus *Calendula officinalis,* Blatt- und/oder Blütenextrakt aus *Euphrasia officinalis,* Blatt- und/oder Blütenextrakt aus *Melilotus officinalis,* Blattextrakt aus *Mentha spicata,* Blattextrakt aus *Mimosa tenuiflora* und Samenextrakt aus *Hordeum vulgare* umfasst.

4. Die Kombination nach einem der Ansprüche 1 bis 3, wobei die Extrakte Wasserextrakte sind.

5. Ein Verfahren zur Herstellung der Kombination wie in einem der Ansprüche 1 bis 4 definiert, wobei das Verfahren die folgenden Schritte umfasst:
(a) Bereitstellen von trockenem Pflanzenmaterial aus *Diffrichia viscosa, Syzygium aromaticum, Vaccinium myrtillus, Calendula officinalis, Euphrasia officinalis, Melilotus officinalis, Mentha spicata, Mimosa tenuiflora,* und *Hordeum vulgare;*
(b) Kochen des trockenen Pflanzenmaterials von (a) zusammen in Wasser, wodurch eine Mischung erhalten wird; und
(c) Destillieren der Mischung von (b), um die Kombination wie in einem der Ansprüche 1-4 definiert zu erhalten;
oder, ersatzweise,
(a) Bereitstellen von trockenem Pflanzenmaterial aus *Diffrichia viscosa, Syzygium aromaticum, Vaccinium myrtillus, Calendula officinalis, Euphrasia officinalis, Melilotus officinalis, Mentha spicata, Mimosa tenuiflora,* und *Hordeum vulgare;*
(b) Kochen des trockenen Pflanzenmaterials von (a) getrennt in Wasser, wodurch eine Vielzahl von Mischungen erhalten wird;
(c) Destillieren der Vielzahl von Mischungen von (b), wodurch eine Vielzahl von Extrakten erhalten wird; und
(c') Kombinieren der Vielzahl von Extrakten von (c), um die Kombination wie in einem der Ansprüche 1 bis 4 definiert zu erhalten;
oder, ersatzweise;
(a) Bereitstellen von trockenem Pflanzenmaterial von *Diffrichia viscosa, Syzygium aromaticum, Vaccinium myrtillus, Calendula officinalis, Euphrasia officinalis, Melilotus officinalis, Mentha spicata, Mimosa tenuiflora,* und *Hordeum vulgare;*
(b) Kochen des trockenen Pflanzenmaterials von (a) getrennt in Wasser, wodurch eine Vielzahl von Mischungen erhalten wird;
(b') Kombinieren der Vielzahl von Mischungen von (b), wodurch eine einzelne Mischung erhalten wird; und
(c) Destillieren der einzelnen Mischung von (b'), um die Kombination wie in einem der Ansprüche 1 bis 4 definiert zu erhalten.

6. Das Verfahren nach Anspruch 5, wobei das trockene Pflanzenmaterial von *Dittrichia viscosa* und *Syzygium aromaticum* in Schritt (a) in einem Gewichtsverhältnis (Gewicht/Gewicht) von 1,5:0,2 bis 1,5:5 bereitgestellt wird; insbesondere in einem Gewichtsverhältnis von etwa 1,5:1.

7. Das Verfahren nach einem der Ansprüche 5 bis 6, wobei trockenes Pflanzenmaterial *von Dittrichia viscosa, Syzygium aromaticum, Vaccinium myrtillus, Calendula officinalis, Euphrasia officinalis, Melilotus officinalis, Mentha spicata, Mimosa tenuiflora, Hordeum vulgare* in Schritt (a) in einem Gewichtsverhältnis von 1,5:0,2:0,3:0,4:0,4:0,4:0,2:0,3:0,4 bis 1,5:5:7,5:10:10:10:5:7,5:10 bereitgestellt werden; insbesondere in einem Gewichtsverhältnis von etwa 1,5:1:1,5:2:2:2:1:1,5:2.

8. Eine Kombination wie in einem der Ansprüche 1 bis 4 definiert, welche durch das Verfahren wie in einem der Ansprüche 5 bis 7 definiert erhalten werden kann.

9. Eine Zusammensetzung umfassend die Kombination wie in einem der Ansprüche 1 bis 4 oder 8 definiert, und wahlweise einen oder mehrere geeignete Hilfsstoffe oder Träger.

10. Die Zusammensetzung nach Anspruch 9, welche eine pharmazeutische Zusammensetzung ist, die eine therapeutisch wirksame Menge der Kombination wie in einem der Ansprüche 1 bis 4 oder 8 definiert, und wahlweise einen oder mehrere pharmazeutisch akzeptable Hilfsstoffe und/oder Träger umfasst;

11. Die Zusammensetzung nach einem der Ansprüche 9 bis 10, welche eine ophthalmische pharmazeutische Zusammensetzung ist, die wahlweise einen oder mehrere ophthalmologisch akzeptable Hilfsstoffe und/oder Träger umfasst.

12. Die Kombination wie in den Ansprüchen 1 bis 4 oder 8 definiert, oder die Zusammensetzung wie in einem der Ansprüche 9 bis 11 definiert, zur Verwendung als Medikament.

13. Die Kombination oder die Zusammensetzung zur Verwendung nach Anspruch 12, welche zur Verwendung bei der Behandlung und/oder Prävention von Presbyopie bestimmt ist.

14. Die Kombination oder die Zusammensetzungen zur Verwendung nach Anspruch 13, wobei die Behandlung und/oder Prävention von Presbyopie die Verabreichung der Kombination oder der Zusammensetzung einmal täglich in jedes Auge umfasst.

15. Die Kombination oder die Zusammensetzungen zur Verwendung nach einem der Ansprüche 13 bis 14,
wobei die Prävention und/oder die Behandlung von Presbyopie Folgendes umfasst:
(a) Verabreichen einer pharmazeutischen Zusammensetzung, umfassend:
eine therapeutisch wirksame Menge an Extrakten aus *Diffrichia viscosa, Syzygium aromaticum, Vaccinium myrtillus, Calendula officinalis, Euphrasia officinalis, Melilotus officinalis, Mentha spicata, Mimosa tenuiflora,* und *Hordeum vulgare;* und
wahlweise einen oder mehrere pharmazeutisch akzeptable Hilfsstoffe und/oder Träger;
oder ersatzweise,
(b) gleichzeitige, aufeinanderfolgende oder getrennte Verabreichung von:
(b1) einer pharmazeutischen Zusammensetzung umfassend eine therapeutisch wirksame Menge eines Extrakts aus *Diffrichia viscosa;* und wahlweise einen oder mehrere pharmazeutisch akzeptable Hilfsstoffe und/oder Träger;
(b2) einer pharmazeutischen Zusammensetzung umfassend eine therapeutisch wirksame Menge eines Extrakts aus *Syzygium aromaticum*; und wahlweise einen oder mehrere pharmazeutisch akzeptable Hilfsstoffe und/oder Träger;
(b3) einer pharmazeutischen Zusammensetzung umfassend eine therapeutisch wirksame Menge eines Extrakts aus *Vaccinium myrtillus*; und wahlweise einen oder mehrere pharmazeutisch akzeptable Hilfsstoffe und/oder Träger;
(b4) einer pharmazeutischen Zusammensetzung umfassend eine therapeutisch wirksame Menge eines Extrakts aus *Calendula officinalis*; und wahlweise einen oder mehrere pharmazeutisch akzeptable Hilfsstoffe und/oder Träger;
(b5) einer pharmazeutischen Zusammensetzung umfassend eine therapeutisch wirksame Menge eines Extrakts aus *Euphrasia officinalis*; und wahlweise einen oder mehrere pharmazeutisch akzeptable Hilfsstoffe und/oder Träger;
(b6) einer pharmazeutischen Zusammensetzung umfassend eine therapeutisch wirksame Menge eines Extraktes aus *Melilotus officinalis*; und wahlweise einen oder mehrere pharmazeutisch akzeptable Hilfsstoffe und/oder Träger;
(b7) einer pharmazeutischen Zusammensetzung umfassend eine therapeutisch wirksame Menge eines Extrakts aus *Mentha spicata;* und wahlweise einen oder mehrere pharmazeutisch akzeptable Hilfsstoffe und/oder Träger;
(b8) einer pharmazeutischen Zusammensetzung umfassend eine therapeutisch wirksame Menge eines Extraktes aus *Mimosa tenuiflora*; und wahlweise einen oder mehrere pharmazeutisch akzeptable Hilfsstoffe und/oder Träger; und
(b9) einer pharmazeutischen Zusammensetzung umfassend eine therapeutisch wirksame Menge eines Extraktes aus *Hordeum vulgare;* und wahlweise einen oder mehrere pharmazeutisch akzeptable Hilfsstoffe und/oder Träger.

## Revendications

1. Une combinaison comprenant des extraits de *Dittrichia viscosa, Syzygium aromaticum, Vaccinium myrtillus, Calendula officinalis, Euphrasia officinalis, Melilotus officinalis, Mentha spicata, Mimosa tenuiflora,* et *Hordeum vulgare.*

2. La combinaison selon la revendication 1, qui comprend au moins l'un des suivants :
- extrait de feuilles de *Dittrichia viscosa* ;
- extrait de bourgeons de fleur de *Syzygium aromaticum* ;
- extrait de feuilles de *Vaccinium myrtillus* ;
- extrait de fleurs de *Calendula officinalis* ;
- extrait de feuilles et/ou de fleurs de *Euphrasia officinalis* ;
- extrait de feuilles et/ou de fleurs de *Melilotus officinalis* ;
- extrait de feuilles de *Mentha spicata* ;
- extrait de feuilles de *Mimosa tenuiflora* ; et
- extrait de graines de *Hordeum vulgare.*

3. La combinaison selon l'une quelconque des revendications 1 à 2, qui comprend : extrait de feuilles de *Dittrichia viscosa,* extrait de bourgeons de fleur de *Syzygium aromaticum,* extrait de feuilles de *Vaccinium myrtillus,* extrait de fleurs de *Calendula officinalis,* extrait de feuilles et/ou de fleurs d*'Euphrasia officinalis,* extrait de feuilles et/ou de fleurs de *Melilotus officinalis,* extrait de feuilles de *Mentha spicata,* extrait de feuilles de *Mimosa tenuiflora* et extrait de graines de *Hordeum vulgare.*

4. La combinaison selon l'une quelconque des revendications 1 à 3, dans laquelle les extraits sont des extraits d'eau.

5. Un procédé de production de la combinaison telle que définie dans l'une quelconque des revendications 1 à 4, le procédé comprenant les étapes consistant à :
(a) fournir de la matière végétale sèche de *Dittrichia viscosa, Syzygium aromaticum, Vaccinium myrtillus, Calendula officinalis, Euphrasia officinalis, Melilotus officinalis, Mentha spicata, Mimosa* Tenuiflora, et *Hordeum vulgare;*
(b) faire bouillir la matière végétale sèche de (a) toute ensemble dans de l'eau, obtenant ainsi un mélange ; et
(c) distiller le mélange de (b) de manière à obtenir la combinaison telle que définie dans l'une quelconque des revendications 1 à 4 ;
ou, en variante,
(a) fournir de la matière végétale sèche de *Dittrichia viscosa, Syzygium aromaticum, Vaccinium myrtillus, Calendula officinalis, Euphrasia officinalis, Melilotus officinalis, Mentha spicata, Mimosa tenuiflora,* et *Hordeum vulgare;*
(b) faire bouillir la matière végétale sèche de (a) séparément dans de l'eau, obtenant ainsi une pluralité de mélanges ;
(c) distiller la pluralité de mélanges de (b), obtenant ainsi une pluralité d'extraits ; et
(c') combiner la pluralité d'extraits de (c) de manière à obtenir la combinaison telle que définie dans l'une quelconque des revendications 1 à 4 ;
ou, en variante ;
(a) fournir de la matière végétale sèche de *Dittrichia viscosa, Syzygium aromaticum, Vaccinium myrtillus, Calendula officinalis, Euphrasia officinalis, Melilotus officinalis, Mentha spicata, Mimosa tenuiflora* et *Hordeum vulgare* ;
(b) faire bouillir la matière végétale sèche de (a) séparément dans de l'eau, obtenant ainsi une pluralité de mélanges ;
(b') combiner la pluralité de mélanges de (b), obtenant ainsi un mélange unique ; et
(c) distiller le mélange unique de (b') de manière à obtenir la combinaison telle que définie dans l'une quelconque des revendications 1 à 4.

6. Le procédé selon la revendication 5, dans lequel la matière végétale sèche de *Dittrichia viscosa* et de *Syzygium aromaticum* sont fournies à l'étape (a) dans un rapport pondéral (poids/poids) de 1,5:0,2 à 1,5:5 ; en particulier, dans un rapport pondéral d'environ 1,5:1.

7. Le procédé selon l'une quelconque des revendications 5 à 6, dans lequel de la matière végétale sèche de *Dittrichia viscosa, Syzygium aromaticum, Vaccinium myrtillus, Calendula officinalis, Euphrasia officinalis, Melilotus officinalis, Mentha spicata, Mimosa tenuiflora, Hordeum vulgare* sont fournies dans l'étape (a) dans un rapport pondéral de 1,5:0,2:0,3:0,4:0,4:0,4:0,2:0,3:0,4 à 1,5:5:7,5:10:10:10:5:7,5:10; en particulier, dans un rapport pondéral d'environ 1,5:1:1,5:2:2:2:1:1,5:2.

8. Une combinaison telle que définie dans l'une quelconque des revendications 1 à 4, qui peut être obtenue par le procédé tel que défini dans l'une quelconque des revendications 5 à 7.

9. Une composition comprenant la combinaison telle que définie dans l'une quelconque des revendications 1 à 4 ou 8, et facultativement un ou plusieurs excipients ou véhicules appropriés.

10. La composition selon la revendication 9, qui est une composition pharmaceutique comprenant une quantité thérapeutiquement efficace de la combinaison telle que définie dans l'une quelconque des revendications 1 à 4 ou 8, et facultativement un ou plusieurs excipients et/ou véhicules pharmaceutiquement acceptables ;

11. La composition selon l'une quelconque des revendications 9 à 10, qui est une composition pharmaceutique ophtalmique, comprenant facultativement un ou plusieurs excipients et/ou véhicules ophtalmiques acceptables.

12. La combinaison telle que définie dans les revendications 1 à 4 ou 8, ou la composition telle que définie dans l'une quelconque des revendications 9 à 11 destinée à être utilisée en tant que médicament.

13. La combinaison ou la composition pour l'utilisation selon la revendication 12, qui est destinée à être utilisée dans le traitement et/ou la prévention de la presbytie.

14. La combinaison ou les compositions pour l'utilisation selon la revendication 13, où le traitement et/ou la prévention de la presbytie comprend l'administration de la combinaison ou de la composition une fois par jour dans chaque œil.

15. La combinaison ou les compositions pour l'utilisation selon l'une quelconque des revendications 13 à 14,
dans lequel la prévention et/ou le traitement de la presbytie comprend :
(a) administrer une composition pharmaceutique comprenant :
une quantité thérapeutiquement efficace d'extraits de *Dittrichia viscosa, Syzygium aromaticum, Vaccinium myrtillus, Calendula officinalis, Euphrasia officinalis, Melilotus officinalis, Mentha spicata, Mimosa tenuiflora,* et *Hordeum vulgare;* et
facultativement, un ou plusieurs excipients et/ou véhicules pharmaceutiquement acceptables ;
ou, en variante,
(b) administrer simultanément, séquentiellement ou séparément :
(b1) une composition pharmaceutique comprenant une quantité thérapeutiquement efficace d'un extrait de *Dittrichia viscosa* ; et facultativement, un ou plusieurs excipients et/ou véhicules pharmaceutiquement acceptables ;
(b2) une composition pharmaceutique comprenant une quantité thérapeutiquement efficace d'un extrait de *Syzygium aromaticum* ; et facultativement, un ou plusieurs excipients et/ou véhicules pharmaceutiquement acceptables ;
(b3) une composition pharmaceutique comprenant une quantité thérapeutiquement efficace d'un extrait de *Vaccinium myrtillus* ; et facultativement, un ou plusieurs excipients et/ou véhicules pharmaceutiquement acceptables ;
(b4) une composition pharmaceutique comprenant une quantité thérapeutiquement efficace d'un extrait de *Calendula officinalis* ; et facultativement, un ou plusieurs excipients et/ou véhicules pharmaceutiquement acceptables ;
(b5) une composition pharmaceutique comprenant une quantité thérapeutiquement efficace d'un extrait *d'Euphrasia officinalis* ; et facultativement, un ou plusieurs excipients et/ou véhicules pharmaceutiquement acceptables ;
(b6) une composition pharmaceutique comprenant une quantité thérapeutiquement efficace d'un extrait de *Melilotus officinalis* ; et facultativement, un ou plusieurs excipients et/ou véhicules pharmaceutiquement acceptables ;
(b7) une composition pharmaceutique comprenant une quantité thérapeutiquement efficace d'un extrait de *Mentha spicata* ; et facultativement, un ou plusieurs excipients et/ou véhicules pharmaceutiquement acceptables ;
(b8) une composition pharmaceutique comprenant une quantité thérapeutiquement efficace d'un extrait de *Mimosa tenuiflora* ; et facultativement, un ou plusieurs excipients et/ou véhicules pharmaceutiquement acceptables ; et
(b9) une composition pharmaceutique comprenant une quantité thérapeutiquement efficace d'un extrait de *Hordeum vulgare* ; et facultativement, un ou plusieurs excipients et/ou véhicules pharmaceutiquement acceptables.
